# EUROPEAN PATENT APPLICATION

(11) **EP 1 658 845 A1**
(43) Date of publication of application: **24.05.2006**
(21) Application number: 04077770.8
(22) Date of filing: 06.10.2004
(51) Int. Cl.: A61K 31/366, A61P 31/18

(54) **Use of statin or precursor thereof for the treatment of virusinfections**

(71) Applicant: PrimaGen Holding B.V., 1105 BA Amsterdam (NL)
(72) Inventor: de Baar, Marinus Petrus, 1011 TB Amsterdam (NL); Paxton, William Anderson, 1011 TB Amsterdam (NL); Pollakis, Georgios, 1011 TB Amsterdam (NL)
(74) Representative: Winckels, Johannes Hubertus F.

(57) **Abstract**

The invention relates to a new use of statins. It was found that statins are capable of reducing human immunodeficiency virus (HIV) infection of cells that are normally susceptible to HIV-infection. Provided are pharmaceuticals, methods and uses of a statin in the treatment of infections.

## Description

The invention relates to the fields of virus infection and immune system modulators.

Currently over 40 million individuals are infected with the human immunodeficiency virus type 1 (HIV-1) world-wide with an estimated 30 million AIDS related deaths to date. Drug intervention strategies have been highly successful in reducing HIV transmission and preventing disease progression, however, they prove expensive and have limitations based on toxicity and drug resistance. Following the successful introduction of nucleoside and non-nucleoside reverse trancriptase inhibitors, protease inhibitors and fusion inhibitors other classes of drugs are currently under development including those that target the virus, such as integrase inhibitors, or the host through targeting HIV-1 receptors at the cell surface. New cheaper drug intervention strategies are urgently needed to stem the rise of new HIV-1 infections in the regions of the world where cost is a restricting factor.

A multitude of CC- and CXC- chemokine receptors can function as viral co-receptors, in conjunction with the CD4 molecule, to enable HIV-1 entry into target cells [1]. The CC-chemokine receptor, CCR5, and the CXC-chemokine receptor, CXCR4, are the two most significant with respect to HIV-1 transmission and pathogenesis [2]. Viruses utilizing the CCR5 receptor (R5) are preferentially transmitted and found during the early stages of infection whilst the CXCR4-using viruses (X4) are those more likely associated with later stage disease [2-4].

The CC-chemokines RANTES, MIP-1α and MIP-1β are the natural ligands for the CCR5 receptor, whilst the CXC-chemokine SDF-1 serves as the ligand for CXCR4 [1]. These molecules have been shown to efficiently block viral entry and suppress replication of the appropriate HIV-1 strains *in vitro.* The R5 viral infectivity profiles can be modulated by CCR5 expression at the cell surface as well as cellular secretion profiles of the CC-chemokines [5, 6]. A number of clinical studies highlight the ability of chemokine and chemokine receptor expression levels to affect viral replication, with low receptor expression and higher chemokine production levels favouring reduced viral replication or even protection from infection [7,8]. Some studies, however, indicate that high CC-chemokine levels enhance viral infectivity in vitro or associate with more rapid progression to disease [9, 10]. The most compelling evidence linking chemokine and chemokine receptor parameters with disease progression is the association of polymorphisms within the genes coding for these molecules with either protection from infection or altered rates of disease progression [4]. Most striking is the 32bp deletion within the coding region of the CCR5 co-receptor gene, D32CCR5, which is associated with a slower rate of progression to disease [2]. It has also been demonstrated that CD4 lymphocytes isolated from individuals pre-infection with HIV can have *in vitro* secretion levels of RANTES from isolated CD4 lymphocytes inversely correlate with HIV-1 viral load measurements post-infection, suggesting that expression levels of CC-chemokines and the corresponding CCR5 co-receptor can be associated with in vivo HIV-1 replication [11]. These results lead to the hypothesis that intervention therapy that is aimed at lowering the CCR5 expression levels or heighten CC-chemokine expression or secretion should positively influence the course of disease progression.

CC-chemokines are involved with the induction of inflammatory responses within the host as well as possessing immunomodulatory functions. It has been demonstrated that CC-chemokine levels measured *in vivo* or *ex vivo* provide accurate correlates of protection elicited by anti-SIV vaccines in non-human primate models. Lehner et al. has previously documented an increased production of RANTES, MIP-1α and MIP-1β by lymph-node CD8⁺ T cells in macaques immunized by the targeted-iliac-lymph-node route [12]. CC-chemokines, by virtue of their unique immunomodulatory properties, may represent a new class of adjuvants for vaccines, capable of directing protective immune responses by recruiting and activating specific cells at the site of immunization. It has been suggested that chemokines play a pivotal role in the Th1/Th2 polarization of CD4⁺ and CD8⁺ T cell cytokine-secretion patterns, a likely significant determinant of vaccine efficacy [13]. For example, protective responses against viruses are believed to require Th1 polarization, which is linked to interferon production and vigorous CTL induction. Therefore regulating CC-chemokine responses may have a significant effect on directing the immune responses mounted against an immunogen and may play an "adjuvant" like role in vaccination protocols.

In the present invention it is demonstrated that statins, traditionally used to lower cholesterol levels in case of hypercholesterolemia, can successfully modulate cell surface expression levels of the CCR5 molecule and secretion levels of the CC-chemokine RANTES. Incubation of CD4 lymphocytes with these compounds had the effect of reducing their in vitro infectivity with CCR5 using viruses but not with CXCR4 using viruses. The effect of incubating CD4 lymphocytes with these statins had the consequences of mimicking the phenotype associated with the heterozygote Δ32CCR5 genotype, demonstrating that statins have the capacity to reduce *in vivo* viral replication and thereby reduce both HIV-1 transmission and rate of disease progression. Thus the invention provides the use of a statin for reducing human immunodeficiency virus infection of cells that are normally susceptible to HIV-infection. The invention further provides the use of a statin for the preparation of a medicament for at least in part reducing human immunodeficiency virus (HIV) infection of cells that are normally susceptible to HIV-infection. HIV viruses can be divided into roughly three groups on the basis of co-receptor usage. Viruses that use receptor CCR5, those that use CXCR4 and those that can utilize both receptors. A statin of the invention inhibits infection of HIV viruses that (partly) utilize the CCR5 receptor for cellular entry, at least in part. Thus in a preferred embodiment said cells express the CCR5 receptor. Apart from the two major co-receptors for HIV infection there are a number of other receptors that have been reported to allow entry of particular strains of HIV into the target cell. For the present invention, these viruses are similar to the viruses that utilise the CXCR4 co-receptor. For such a virus, entry can be dependent on the presence of the minor co-receptor or not. In other words, if the virus dependents exclusively on the minor co-receptor it is not likely that virus entry can be at least in part inhibited through statins. However, more often, such viruses can utilize more than one co-receptor including CCR5 like, for instance, HIV viruses that can utilize both CXCR4 and CCR5. If in the case of multiple co-receptor usage one of the possible co-receptors is CCR5, statins are effective in at least in part inhibiting virus infection. Considering that the effectiveness of statins in at least in part inhibiting HIV infection depends on the type of HIV that infects the cells and the fact that many different HIV types exist, it is not possible to give a definitive level of inhibition. Even more so because HIV samples, particularly when obtained from patients, typically consist of a mixture of different strains of HIV that may differ considerably in their co-receptor usage. HIV viruses are currently divided in two major groups, HIV-1 and HIV-2. Any virus is inhibited as long as the particular HIV virus utilizes the co-receptor CCR5 for entry into the target cell. For instance, it is possible to generate chimearic viruses having the cell entry proteins of HIV and other (capsid) proteins of a different virus. Chimearic viruses comprising at least the gp120 protein of HIV are capable of entering a HIV susceptible cell in much the same way as a wild-type HIV. HIV is currently also investigated as a gene transfer vehicle or as a vaccine. Such gene transfer vehicles or vaccines typically differ from wild type HIV in that some function of wild type HIV is altered or deleted. Statins can also inhibit infection of such vehicles or vaccines when they utilize the CCR5 co-receptor for entry into the cell. Thus, the invention provides a method for at least in part inhibiting cellular entry of a virus like particle wherein said particle comprises at least one HIV derived protein that allows usage of the co-receptor CCR5 for entry of the virus like particle into the target cell. A protein that determines co-receptor usage is gp120 of HIV. In preferred embodiment the HIV is HIV-1.

Various cell types are susceptible to HIV infection. A susceptible cell typically expresses human CD4. However, it has been shown possible to infect cells from other species with HIV as long as the species is not to distant in evolutionary terms from the human species. One species that allows infection by HIV is the chimpanzee. The CD4 molecule of a chimpanzee is therefore functionally equivalent to a CD4 molecule of a human. Thus a susceptible cell preferably expresses a human CD4 molecule or a functional equivalent thereof. The functional equivalent can be a CD4 molecule of a chimpanzee, and/or a part of a CD4 molecule sufficient to allow infection of a cell otherwise containing all the essential proteins for viral entry. A functional equivalent can also be a derivative of a CD4 molecule. Such derivatives comprises the same HIV virus receptor function in kind but not necessarily in amount as human CD4. Thus murine cells provided with a human CD4 molecule are for the present invention also regarded as susceptible cells. In a preferred embodiment the susceptible cell is a human or chimpanzee cell. Cells from these species closely resemble normal cells of a human individual that is to be treated with a statin according to a method or use of the invention.

In the present invention it was found that HIV infection of CD4⁺ cells can be inhibited by providing the cells with statins. A statin is herein defined as a substance capable of inhibiting the enzyme HMG CoA reductase. The HMG CoA reductase enzyme plays a role in cholesterol synthesis and statins are often given to individuals suffering from elevated levels of cholesterol. We have identified that incubation of CD4+ enriched lymphocytes with statins reduces the infectivity with CCR5 using but not CXCR4 using HIV-1 viruses. Without being bound to theory it is believed that statins act through reducing the amount of CCR5 that is available for the virus to facilitate entry. The cells were obtained from normal individuals that were not treated otherwise. Statins were shown to have the effect of modulating CCR5 and RANTES expression levels in a manner that favours the inhibition of CCR5 virus replication. The treatment of the cells with these compounds has the effect of down-modulating CCR5 expression at the cell surface whilst increasing the expression levels of the CC-chemokine RANTES. Both of these characteristics have previously been associated with CD4+ lymphocytes isolated from individuals heterozygous for the Δ32bp deletion in the CCR5 gene and which also are refractory for replication of CCR5 using viruses. Individuals heterozygous for the Δ32bp deletion in the CCR5 gene have been shown to harbour lower viral loads and progress slower in their disease course than individuals homozygous for the CCR5 wild-type gene. A number of other genetic linkages have been associated with CCR5 and RANTES and which correlate with altered rates of disease progression [7, 8]. Collectively the association between chemokine and chemokine receptor genotypes suggests that administration of a statin reduces HIV-1 viral loads and rates of disease progression. In any case, whatever the underlying technical reason, our results indicate that treating individuals with statins is beneficial for individuals at risk of acquiring HIV or HIV infected individuals. Particularly for individuals carrying and normally expressing CCR5.

The HIV infection inhibiting effect of statins that was observed in the present invention is not necessarily linked with the HMG CoA reductase inhibiting effect of statins. Some precursors of statins that need to be metabolised into an active statin in a cell are as effective in inhibiting HIV-infection of otherwise susceptible cells as a statin that does not need to be metabolised for HMG CoA reductase inhibition. Thus the invention further provides the use of a statin precursor that after entry into a cell is metabolised into the active statin, for at least in part reducing human immunodeficiency virus (HIV) infection of cells that are normally susceptible to HIV-infection. The invention further provides the use of said precursor for the preparation of a medicament for at least in part reducing human immunodeficiency virus (HIV) infection of cells that are normally susceptible to HIV-infection. Also provided is the use of said precursor in a method of the invention. The activated statin may exert its effect in the cell wherein it is metabolised but may also migrate out of the cell to exert its effect on a different cell. In a preferred embodiment said statin comprises simvastatin, lovastatin or mevastatin, or a precursor thereof.

Statins are not only effective in at least in part inhibiting infection by HIV as described above, but also in reducing the speed with which the virus spreads to uninfected cells. In one embodiment statin or a precursor thereof is used for the preparation of a medicament for the treatment of an HIV-infected individual or an individual at risk of acquiring an HIV-infection. Since statin or precursors thereof act on the level of infectivity they work synergistically with HIV compounds that act on a different level such as nucleotide analogues and other reverse transcriptase inhibitors, protease inhibitors etc. Thus in a preferred embodiment said individual is receiving further medication for the treatment of said HIV-infection.

CCR5 dependent viruses prevail very early in infection. Later in infection and particularly when the immune system is on the brink of collapse, HIV viruses exhibiting different co-receptor preferences become more prominent. In a preferred embodiment a use or method of the invention is for the treatment of HIV-infected individuals for whom the diagnosis AIDS has not been given or who have a mean CD4 count above 500. Preferably, the statin is used or said medicament is prepared for individuals who are within three years from seroconverting for HIV specific antibodies. Statins or precursors thereof are most effective early after exposure to the virus up until three years from infection. As CCR5 using viruses are also present after this date, statins or precursors thereof are still effective, particularly when the individual was on HIV medication in the first three years. Therapy in that period on average significantly reduces the speed with which viruses appear that have co-receptor usage other than CCR5. In a preferred embodiment a use or method of the invention is for the treatment of individuals who are within one year from seroconverting for HIV specific antibodies.

In HIV-infected patients, statins or precursors thereof are effective in reducing viral load of HIV patients that are otherwise untreated. Statins or precursors thereof also prolong life expectancy in such patients and further prolong the disease-free period prior to the development of AIDS-like symptoms. In combination therapy with a medicament acting on a different level, statins or precursors thereof result in a rise in the CD4 count in the treated individuals but also prolong disease free survival and reduce viral load below a detection level of 1000 particles per ml. In a preferred embodiment the statin is used for the preparation of a medicament for at least in part preventing the infection of a newborn from an HIV-infected woman. In one aspect of this preferred embodiment said use is for reducing infection and viral load in the HIV-infected woman. In a preferred aspect of this embodiment the medicament is given to at least reduce the chance of infection of the newborn with HIV virus of the mother.

In another aspect the invention provides a method for augmenting an immune response in a subject comprising providing said subject with a statin or a precursor thereof. The immune system is sometime divided into a Th1 and a Th2-type of response. An immune response directed toward a pathogen is typically composed of a Th1 and a Th2-type response. The amount with which each type of response is initiated or boosted differs between pathogens but also between individuals. The ratio between the Th1 and the Th2-type of response is referred to as the Th1/Th2 balance. Through action on the CCR5 receptor and particularly through its effect on the expression of RANTES in blood cells from normal individuals statins or precursors thereof are capable of raising the Th1 response compared to the Th2 response, i.e. shifting the Th1/Th2 balance in favour of a Th1 type response. This property is used in the present invention to modulate an immune response in an individual. Thus the invention provides the use of a statin or precursor thereof wherein said statin or precursor thereof has an immune modulator effect, preferably through its effect on the expression of RANTES. Heightened expression of RANTES is associated with directing a Th1 skewing and predictably aids in the induction of more efficient immune responses against viral infections, such as HIV-1 [12, 13]. This property of the use of a statin or precursor thereof according to the invention can also be used in a different setting than HIV-infected individuals or individuals at risk of acquiring the disease. The onset of many auto-immune diseases and, for that matter, allergies is characterised by a more Th2-type of response towards the auto-antigen or allergen. In the present invention a statin or precursor thereof is used to at least in part shift the Th1/Th2 balance more towards a more suitable Th1 type of response for those diseases. A more Th-1 type response is also favoured for immunizations and vaccinations. Thus the invention further provides the use of a statin or precursor thereof as an adjuvant in a vaccine or immunization preparation. In another embodiment the invention provides the use of a statin or precursor thereof for the preparation of a composition for improving an immune response against an immunogenic compound. Statins are typically given to patients in an oral formulation. In the present invention it is preferred that the statin or precursor thereof is provided in the immunizing or vaccine composition. This may be followed up by a suitable number of oral administrations over the next days for further augmentation. In a preferred embodiment, a composition of the invention comprises an immunogenic compound, preferably present in a vaccine.

### EXAMPLES

### Example 1

CD4⁺ enriched lymphocytes were prepared by the following protocol. PBMCs were isolated from fresh buffy coats (Sanquin, Amsterdam) by standard Ficoll-Hypaque density centrifugation and frozen in multiple vials at high concentrations, thawed when required and activated with 2 µg/ml PHA (sigma) and cultured in RPMI media containing 10% FCS, Penicillin (100 U/ml) and Streptomycin (100 U/ml) with recombinant IL-2 (100 U/ml). On day 3 of culture the cells underwent CD4⁺ enrichment by removing CD8⁺ lymphocytes with CD8 immunomagnetic beads and magnet (Dynal, Oslo, Norway), giving rise to cultures of > 90% CD4⁺ lymphocyte purity.

Enriched CD4⁺ lymphocytes were cultured for 72 hours either in the presence of 0.5 or 5.0µM of each statin independently (simvastatin, lovastatin, mevastatin) or in the presence of media alone. The cells were then counted and subsequently incubated at a set live concentration in fresh media (1.0x10⁶ live cells/ml). The cells were split and infected with either a CCR5 using virus isolate (SF162) or a CXCR4 using virus isolate (SF2) both at a 1,000 TCID₅₀/ml of 1,000 (m.o.i = 0.001) and in the presence of the appropriate statin or media alone. After an incubation period of 3 hours the cells were washed, counted and re-plated. The infected cells were plated either undiluted (2.0x10⁵ cells/well) or in 2-fold dilutions with an additional 2.0x10⁵ fresh CD4⁺ lymphocytes added to each well in fresh media. The culture supernatants were harvested and refreshed on days 7 and 14 with virus replication monitored by measuring CA-p24 levels utilizing a standard ELISA protocol.

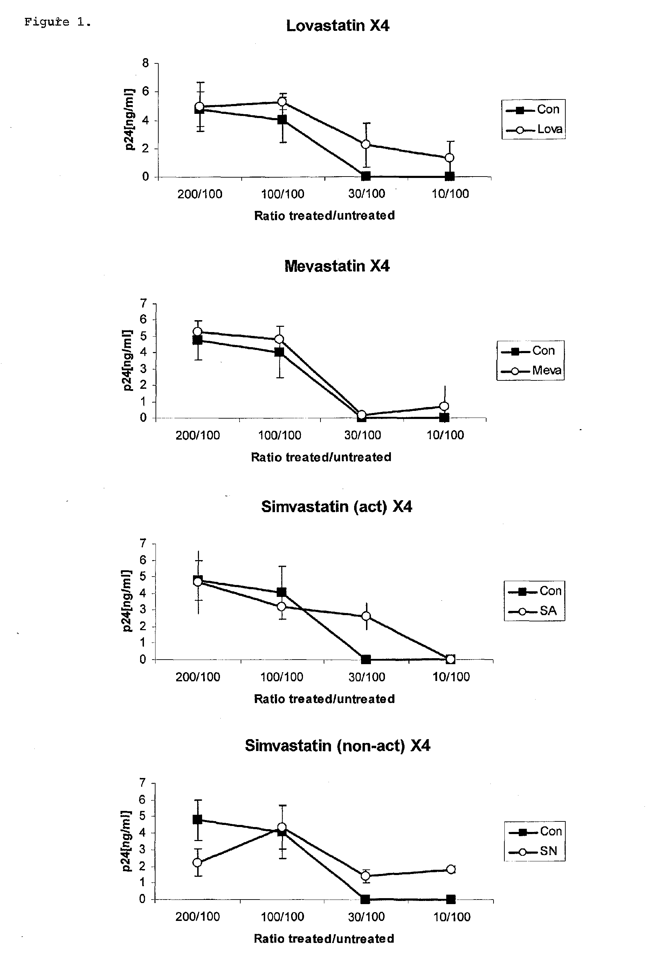

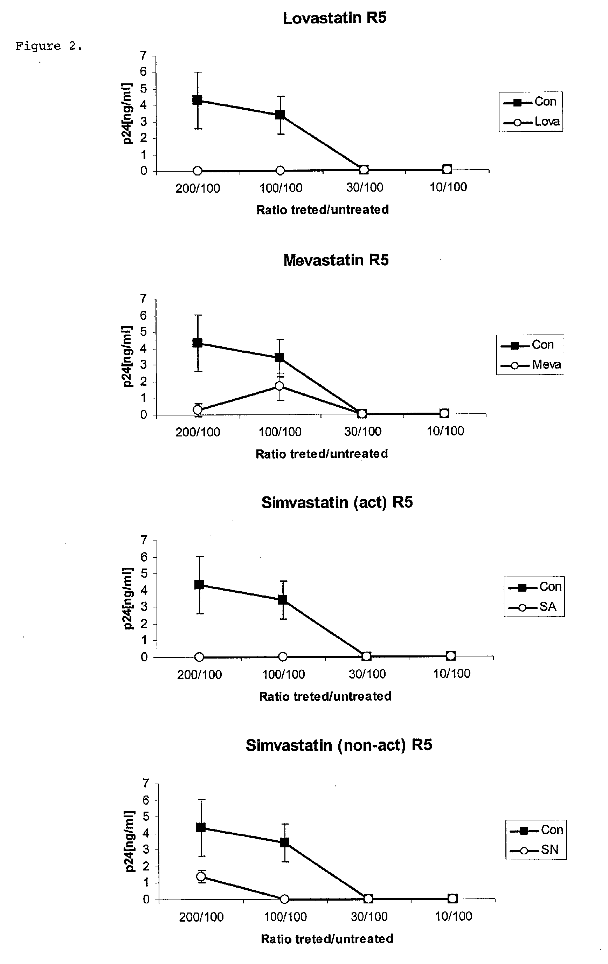

No variation was observed between cells treated with the statins versus the control cells for the CXCR4 using virus (Fig. 1). Large differences in infectivity were observed with the CCR5 using virus between the control cells and cells treated with the statins (Fig 2). The similar replication capacity of the CXCR4 using virus on the control cells as well as the statin treated cells indicates that the effect observed for the CCR5 using virus is not due to cytotoxic or cytosolic effects. Cell counts and cell viabilities were performed on day 4 of incubation with statins and no differences were observed between these and the control cells incubated with media alone (data not shown), again supporting the interpretation that the statin effect on the CD4+ lymphocytes is not due to cytotoxic or cytosolic effects of the drugs.

### LEGENDS TO THE FIGURES

### Figure 1

Antigen p24 measured in cell cultures as a measure of CXCR4 using HIV-1 replication with different statins added to the culture. In each panel Con means control cells to which no statins were added. The ratio treated/untreated reflects the ratio between cells infected with the virus and cells not infected with the virus in the last culture step to propagate any virus that entered the cells during the incubation of cells with virus (see example 1 for further explanation of culture order).

### Figure 2

Antigen p24 measured in cell cultures as a measure of CCR5 using HIV-1 replication with different statins added to the culture. In each panel Con means control cells to which no statins were added. The ratio treated/untreated reflects the ratio between cells infected with the virus and cells not infected with the virus in the last culture step to propagate any virus that entered the cells during the incubation of cells with virus (see example 1 for further explanation of culture order).

### REFERENCES

1. Unutmaz D, KewalRamani VN, Littman DR. G protein-coupled receptors in HIV and SIV entry: new perspectives on lentivirus-host interactions and on the utility of animal models. Seminars in Immunology 1998;10(3):225-36.
2. Paxton WA, Kang S. Chemokine receptor allelic polymorphisms: relationships to HIV resistance and disease progression. Seminars in Immunology 1998;10(3):187-94.
3. Michael NL, Chang G, Louis LG, Mascola JR, Dondero D, Birx DL, Sheppard HW. The role of viral phenotype and CCR-5 gene defects in HIV-1 transmission and disease progression. Nature Med. 1997;3:338-40.
4. Michael NL. Host genetic influences on HIV-1 pathogenesis. Curr.Opin.Immunol. 1999;11(4):466-74.
5. Paxton WA, Liu R, Kang S, Wu L, Gingeras TR, Landau NR, Mackay CR, Koup RA. Reduced HIV-1 infectability of CD4+ lymphocytes from exposed-uninfected individuals: association with low expression of CCR5 and high production of beta-chemokines. Virology 1998;244(1):66-73.
6. Wu L, Paxton WA, Kassam N, Ruffing N, Rottman JB, Sullivan N, Choe, H, Sodroski J, Newman W, et al. CCR5 levels and expression pattern correlate with infectability by macrophage-tropic HIV-1, in vitro. Journal of Experimental Medicine 1997;185(9):1681-91.
7. Zagury D, Lachgar A, Chams V, Fall LS, Bernard J, Zagury JF, Bizzini, B, Gringeri A, Santagostino E, et al. C-C chemokines, pivotal in protection against HIV type 1 infection. Proceedings of the National Academy of Sciences of the United States of America 1998;95(7):3857-61.
8. Saha K, Bentsman G, Chess L, Volsky D. Endogenous production of b-chemokines by CD4+, but not CD8+, T-cell clones correlates with the clinical state of human immunodeficiency virus type 1 (HIV-1)-infected individuals and may be responsible for blocking infection with non-syncytium-inducing HIV-1 in vitro. Journal of Virology 1998;72:876-81.
9. Gordon CJ, Muesing MA, Proudfoot AE, Power CA, Moore JP, Trkola A. Enhancement of human immunodeficiency virus type 1 infection by the CC-chemokine RANTES is independent of the mechanism of virus- cell fusion. Journal of Virology 1999;73(1):684-94.
10. Polo S, Veglia F, Malnati MS, Gobbi C, Farci P, Raiteri R, Sinicco, A, Lusso P. Longitudinal analysis of serum chemokine levels in the course of HIV-1 infection. AIDS 1999;13(4):447-54.
11. Paxton WA, Neuman AU, Kang S, Deutch L, Brown RC, Koup RA, Wolinsky SM. RANTES production from CD4+ lymphocytes correlates with host genotype and rates of human immunodeficiency virus type-1 (HIV-1) disease progression. Journal of Infectious Diseases 2001; 183(11):1678-1681.
12. Lehner T, Wang Y, Cranage M et al., Protective mucosal immunity elicited by targeted iliac lymph node immunization with a subunit SIV envelope and core vaccine in macaques. Nature Medicine 1996; 2:767-775.
13. Lusso P. HIV and chemokines: Implications for therapy and vaccine. Vaccine 2002;15:1964-1967.

## Claims

1. Use of a statin or precursor thereof for the preparation of a medicament for at least in part reducing human immunodeficiency virus (HIV) infection of cells that are normally susceptible to HIV-infection.

2. Use according to claim 1, wherein said cell is a cell that normally expresses CCR5.

3. Use according to claim 1 or claim 2, wherein said statin or precursor thereof comprises symvastatin or precursor thereof, lovastatin or precursor thereof or mevastatin or precursor thereof.

4. Use of a statin or precursor thereof for the preparation of a medicament for the treatment of an HIV-infected individual or an individual at risk of acquiring an HIV infection.

5. Use according to claim 4, wherein said individual is receiving further medication for the treatment of said HIV-infection.

6. Use according to claim 4 or claim 5, for the treatment of HIV-infected individuals for whom the diagnosis AIDS has not been given or who have a mean CD4 count above 500.

7. Use according to any one of claims 4-6, wherein said medicament is prepared for individuals who are within three years from seroconverting for HIV specific antibodies.

8. Use according to any one of claims 4-7, wherein said medicament is prepared for individuals who are within one year from seroconverting for HIV specific antibodies.

9. Use of a statin or precursor thereof for the preparation of a medicament for at least in part preventing the infection of a newborn from an HIV-infected woman.

10. Use according to claim 9, wherein said medicament is prepared for the treatment of the HIV-infected woman.

11. Use according to claim 9, wherein said medicament is prepared for the treatment of the newborn.

12. A method for augmenting an immune response in a subject comprising providing said subject with a statin or precursor thereof.

13. Use of a statin or precursor thereof according to claim 12, wherein said statin or precursor thereof has an immune modulator effect.

14. Use of a statin or precursor thereof according to claim 12, wherein said statin or precursor thereof has an immune modulator effect through the stimulation of RANTES.

15. Use of a statin or precursor thereof as an adjuvant in a vaccine or immunization preparation.

16. Use of a statin or precursor thereof according to claim 15, wherein said statin or precursor thereof has an immune modulator effect.

17. Use of a statin or precursor thereof according to claim 15, wherein said statin or precursor thereof has an immune modulator effect through the stimulation of RANTES.

18. Use of a statin or precursor thereof for the preparation of a composition for improving an immune response against an immunogenic compound.

19. Use of a statin or precursor thereof according to claim 18, wherein said immunogenic compound is a virus or part thereof.

20. Use of a statin or precursor thereof according to claim 18-19, wherein said immunogenic compound is present in a vaccine.

21. Use of a statin or precursor thereof according to claim 18-20, wherein said statin or precursor thereof has an immune modulator effect.

22. Use of a statin or precursor thereof according to claim 18-20, wherein said statin or precursor thereof has an immune modulator effect through the stimulation of RANTES.

23. A method for at least in part reducing human immunodeficiency virus (HIV) infection of cells that are normally susceptible to HIV-infection comprising providing said cells with a statin or precursor thereof.
